(19) Europäisches Patentamt
European Patent Office
Office européen des brevets

(11) **EP 0 898 974 B1**

(12) **EUROPÄISCHE PATENTSCHRIFT**

(45) Veröffentlichungstag und Bekanntmachung des Hinweises auf die Patenterteilung:
**09.04.2008 Patentblatt 2008/15**

(51) Int Cl.:
***A61M 1/16*** *(2006.01)*

(21) Anmeldenummer: **98115125.1**

(22) Anmeldetag: **12.08.1998**

(54) **Blutbehandlungsvorrichtung mit einer Einrichtung zur Bestimmung von Parametern der Hämodialyse, sowie Verfahren zu ihrer Bestimmung**

Blood treatment apparatus including a device to determine hemodialysis parameters and a method for their determination

Appareil de traitement du sang comprenant un dispositif de determination de paramètres de dialyse et méthode de détermination de paramètres

(84) Benannte Vertragsstaaten:
**DE ES FR GB IT**

(30) Priorität: **13.08.1997 DE 19734992**
**19.02.1998 DE 19806900**

(43) Veröffentlichungstag der Anmeldung:
**03.03.1999 Patentblatt 1999/09**

(60) Teilanmeldung:
**08003419.2**

(73) Patentinhaber: **Fresenius Medical Care Deutschland GmbH**
**61352 Bad Homburg v.d.H. (DE)**

(72) Erfinder:
• **Goldau, Rainer**
**97440 Schraudenbach (DE)**
• **Krämer,Dr.Matthias**
**61381 Friedrichsdorf (DE)**

(74) Vertreter: **Luderschmidt, Schüler & Partner Patentanwälte**
**John-F.-Kennedy-Strasse 4**
**65189 Wiesbaden (DE)**

(56) Entgegenhaltungen:
WO-A-94/08641          DE-A- 3 938 662
US-A- 4 923 613

**Beschreibung**

Die Erfindung betrifft ein Verfahren zur Bestimmung von Parametern der Hämodialyse während einer extrakorporalen Blutbehandlung sowie eine Blutbehandlungsvorrichtung mit einer Einrichtung zur Bestimmung von Parametern der Hämodialyse.

Eine wesentliche Aufgabe der Nieren des Menschen liegt in der Absonderung harnpflichtiger Stoffe aus dem Blut und der Regelung der Wasser- und Elektrolytausscheidung. Die Hämodialyse stellt ein Behandlungsverfahren zur Kompensation von Fehlfunktionen der Nieren bezüglich der Entfernung der harnpflichtigen Stoffe und der Einstellung der Elektrolytkonzentration im Blut dar. Die Wasserausscheidung wird dabei über eine Ultrafiltrationseinrichtung kontrolliert.

Das Blut wird bei der Hämodialyse in einem extrakorporalen Kreislauf durch die Blutkammer eines Dialysators geleitet, die über eine semipermeable Membran von einer Dialysierflüssigkeitskammer getrennt ist. Die Dialysierflüssigkeitskammer wird von einer die Blutelektrolyte in einer bestimmten Konzentration enthaltenen Dialysierflüssigkeit durchströmt. Die Stoffkonzentration ($c_d$) der Dialysierflüssigkeit entspricht der Konzentration des Blutes eines Gesunden. Während der Behandlung wird das Blut des Patienten und die Dialysierflüssigkeit an beiden Seiten der Membran im allgemeinen im Gegenstrom mit einer vorgegebenen Flußrate ($Q_b$ bzw. $Q_d$) vorbeigeführt. Die harnpflichtigen Stoffe diffundieren durch die Membran von der Blutkammer in die Kammer für Dialysierflüssigkeit, während gleichzeitig im Blut und in der Dialysierflüssigkeit vorhandene Elektrolyte von der Kammer höherer Konzentration zur Kammer niedrigerer Konzentration diffundieren. Durch Anlegen eines Transmembrandrucks kann der Stoffwechsel zusätzlich beeinflußt werden.

Um das Blutbehandlungsverfahren optimieren zu können, ist die Bestimmung von Parametern der Hämodialyse während der extrakorporalen Blutbehandlung (in-vivo) notwendig. Von Interesse ist insbesondere der Wert für die Konzentration einer Substanz im Blut am Eingang des Dialysators oder die Austauschleistung des Dialysators für eine bestimmte Substanz, die durch die sogenannte "Clearance" bzw. "Dialysance D" dargestellt wird.

Als Clearance für einen bestimmten Stoff K wird dasjenige virtuelle (errechnete) Blutvolumen bezeichnet, das pro Minute unter definierten Bedingungen im Dialysator vollkommen von einem bestimmten Stoff befreit wird. Die Dialysance ist ein weiterer Begriff zur Bestimmung der Leistungsfähigkeit eines Dialysators, bei dem die Konzentration der eliminierten Substanz in der Dialysierflüssigkeit berücksichtigt wird. Neben diesen Parametern zur Beschreibung der Leistungsfähigkeit des Dialysators sind noch andere Parameter von Bedeutung, wie die Werte des wäßrigen Anteils des Blutes, des Blutvolumens und der Bluteingangskonzentration etc..

Die meßtechnisch-mathematische Quantifizierung der Blutreinigungsverfahren und die Bestimmung der vorgenannten Parameter der Dialyse ist relativ komplex. Hinsichtlich der Berechnungsgrundlagen wird auf Sargent,, J.A., Gotch. F.A.,: Principles and biophysics of dialysis, in: Replacement of Renal Function by Dialysis, W. Drukker, F.M. Parsons, J.F. Maher (Hrsg). Nijhoff, Den Haag 1983 verwiesen.

Die Dialysance bzw. die Clearance kann für einen gegebenen Elektrolyten, beispielsweise Natrium, bei einer Ultrafiltrationsrate von Null wie folgt bestimmt werden. Die Dialysance D ist gleich dem Verhältnis zwischen dem blutseitigen Massentransport für diesen Eletkrolyten ($Q_b$ x (cbi - cbo)) und der Konzentrationsdifferenz dieses Elektrolyten zwischen dem Blut und der Dialysierflüssigkeit am jeweiligen Eingang des Dialysators (cbi - cdi).

$$D = Qb \cdot \frac{cbi-cbo}{cbi-cdi} \qquad (1)$$

Aus Gründen der Massenbilanz gilt:

$$Qb \cdot (cbi-cbo) = -Qd \cdot (cdi-cdo) \qquad (2)$$

Aus den beiden oben genannten Gleichungen (1) und (2) folgt:

$$D = Qd \cdot \frac{cdo-cdi}{cbi-cdi} \qquad (3)$$

Dabei bedeuten in (1) bis (3):

$Q_b$ = effektiver Blutfluß
$Q_d$ = Dialysierflüssigkeitsfluß
cb = Stoffkonzentration im Blut
cd = Stoffkonzentration in der Dialysierflüssigkeit
i = Eingang des Dialysators
o = Ausgang des Dialysators

Der effektive Blutfluß ist der Fluß des Blutanteils, in dem die zu entfernenden Stoffe gelöst sind, d.h., er bezieht sich auf das (wäßrige) Lösungsvolumen für diesen Stoff. Je nach Stoff kann das der Plasmawasserfluß oder der Blutwasserfluß, d.h. der gesamte Wasseranteil im Vollblut sein. Die Konzentration cb bezieht sich auf den entsprechenden Blutanteil.

Für den Fall eines besonderen Stoffwechselausscheidungsprodukts, z.B. Harnstoff, ist cdi = 0, da diese Substanz bestimmungsgemäß in der frischen Dialysierflüssigkeit nicht vorhanden ist. Dann spricht man nicht mehr von der Dialysance, sondern vor der Clearance K für dieses Stoffwechselprodukt.

$$K = Qb\frac{(cbi - cbo)}{cbi} = Qd\frac{cdo}{cbi} \qquad (4)$$

Die bekannten Verfahren zur in-vivo-Bestimmung von Parametern der Hämodialyse basieren auf den obigen Überlegungen. Dabei besteht das Bestreben, ohne einen direkten Meßeingriff in die Blutseite auszukommen, da dieser nämlich eine nicht unerhebliche Gefahrenquelle darstellt. Die zu bestimmenden Größen müssen daher allein aus dialysatseitigen Messungen abgeleitet werden.

Durch die EP 0 291 421 B1 ist ein Verfahren zur Bestimmung der Bluteingangskonzentration cbi bekannt geworden, bei dem rampenförmig die Dialysateingangskonzentration verändert wird, um den Punkt zu bestimmen, wo kein Elektrolyttransfer über die Membran mehr stattfindet. Das bekannte Verfahren arbeitet daher nach dem Prinzip, die Eingangsleitfähigkeit der Dialysierflüssigkeit soweit zu verändern, daß sie sich nicht mehr von der Ausgangsleitfähigkeit unterscheidet. Dann muß sie die Bluteingangsleitfähigkeit angenommen haben (cbi = cdi). Auf der Basis der Gleichungen (1) bis (3) lassen sich dann andere Parameter der Hämodialyse ableiten wie der Wert von D oder Qb. Nachteilig bei diesem Verfahren ist die verhältnismäßig lange Meßzeit, bedingt durch die Zeitspanne bis zum Erreichen des stabilen Gleichgewichtszustandes beim Einstellen der Dialysierflüssigkeit auf den neuen Eingangskonzentrationswert, der sich zudem nicht sofort an jedem Punkt des Dialysators auswirkt. Es dauert systembedingt eine gewisse Zeit, bis ein Leitfähigkeitssprung am Dialysateingang zu stabilen Verhältnissen am Dialysatausgang führt. Die zum Erreichen des stabilen Gleichgewichtszustandes erforderliche Zeitspanne ist dabei im wesentlichen bestimmt von der Größe der Leitfähigkeitsveränderung pro Zeit. Innerhalb dieser langen Zeitspanne können sich jedoch Parameter der Dialyse ändern und somit den zu bestimmenden Wert verfälschen. Insbesondere ist zu beachten, daß das vorgenannte bekannte Verfahren (wie alle anderen auch) direkt die Bluteingangskonzentration cbi durch den herbeigeführten Elektrolyttransfer verändern kann. Im bekannten Fall ist dieser systematische Fehler durch die Art der Veränderung der Konzentration auf der Dialysatseite besonders groß. Das bekannte Verfahren führt damit nicht zu genauen Meßwerten für die in-vivo zu bestimmenden Parameter der Hämodialyse. Hinzu kommt, daß eine relativ aufwendige zusätzliche Vorrichtung zum Variieren der Dialysateingangskonzentration benötigt wird.

Die DE 39 38 662 C2 (EP 0 428 927 A1) beschreibt ein Verfahren zur in-vivo-Bestimmung von Parametern der Hämodialyse, bei dem der Dialysat-Elektrolyttransfer jeweils bei zwei unterschiedlichen Dialysateingangskonzentrationen gemessen wird. Unter der Annahme, daß die Bluteingangskonzentration konstant ist, wird nach dem bekannten Verfahren die Dialysance dadurch bestimmt, daß die Differenz zwischen den Differenzen der Dialysierflüssigkeits-Ionenkonzentration an der Eingangsseite und der Ausgangsseite des Dialysators zum Zeitpunkt der ersten und zweiten Messung bestimmt wird, diese durch die Differenz der Dialysierflüssigkeits-Ionenkonzentration an der Eingangsseite zum Zeitpunkt der ersten Messung und der zweiten Messung geteilt wird und der Quotient hieraus mit dem Dialysierflüssigkeitsfluß multipliziert wird.

Die o. g. Verfahren erweisen sich insofern als nachteilig, als relativ aufwendige Vorrichtungen zum Variieren der Dialysateingangskonzentration erforderlich sind.

Die EP 0 330 892 A1 und die EP 0 547 025 beschreiben ein weiteres Verfahren, das die Bestimmung der Bluteingangskonzentration zum Ziel hat. Bei dem bekannten Verfahren wird die Differenz der Dialysierflüssigkeits-Ionenkonzentration an der Ein- und Ausgangsseite des Dialysators, d.h. die Elektrolyttransferrate, bestimmt. Das bekannte Verfahren arbeitet auch mit unterschiedlichen Konzentrationseinstellungen im Dialysierflüssigkeitsweg, so daß ebenfalls eine relative aufwendige Vorrichtung zum Variieren der Dialysierflüssigkeitseingangskonzentration erforderlich ist.

Aus der US-A-5,110,477 ist ein Verfahren zur Bestimmung der Clearance eines Dialysators bekannt, bei dem sowohl auf der Dialysat- als auch der Blutseite Kalibrierlösungen durch den Dialysator geleitet werden. Auch dieses Verfahren erfordert eine relativ aufwendige Apparatur. Darüber hinaus kann es nicht in-vivo, sondern nur in-vitro, d.h. außerhalb der laufenden Dialysebehandlung, durchgeführt werden.

Die WO 94/09351 beschreibt einen Harnstoffsensor für eine Hämodialysevorrichtung, der zur Analyse dem Dialysierflüssigkeitsweg verbrauchte Dialysierflüssigkeit entnimmt. Die Dialysierflüssigkeitsentnahme setzt eine bestimmte Flußrate der Dialysierflüssigkeit voraus.

Aus dem Artikel Clinical Validation of a Predictive Modeling Equation for Sodium, Autoren: Nguyen-Khoa Man et al.; Herausgeber: Artificial Organs, 9(2): 150 bis 154 (1985), Raven Press, New York ist ein Verfahren zum Bestimmen der Na-Konzentration im Blutkreislauf einer künstlichen Niere bekannt, wobei die Messung im Dialysierflüssigkeitskreislauf erfolgt. Bei dem bekannten Verfahren wird die zum Dialysator führende und vom Dialysator abgehende Leitung des Dialysierflüssigkeitskreislaufs kurzgeschlossen, damit nur eine kleine Menge an Dialysierflüssigkeit von der Dialysierflüssigkeitspumpe durch den Dialysator gepumpt wird. Dadurch wird erreicht, daß sich ein Gleichgewichtszustand einstellt und die Konzentrationswerte im Blutkreislauf denjenigen im Dialysierflüssigkeitskreislauf entsprechen.

Aus der EP 0 578 585 B1 ist ebenfalls ein Verfahren bekannt, bei dem die Messung auf der Dialysierflüssigkeitsseite nach der Einstellung eines Gleichgewichtszustandes erfolgt. Auch bei diesem Verfahren rezirkuliert eine nur kleine Menge an Dialysierflüssigkeit durch den Dialysator, bis sich der Gleichgewichtszustand eingestellt hat.

Die o.g. Verfahren, bei denen während der Messung nur eine kleine Menge an Dialysierflüssigkeit durch den Dialysator geleitet wird, sind insofern nachteilig, als eine Umleitung der Dialysierflüssigkeit im Dialysierflüssigkeitsweg erforderlich ist. Dies ist mit einem zusätzlichen apparativen Aufwand verbunden.

Darüber hinaus sind Hämodialysevorrichtungen bekannt, bei denen stromauf und stromab der Dialysierflüssigkeitskammer des Dialysators Absperrorgane und eine den Strömungsweg durch die Dialysierflüssigkeitskammer des Dialysators überbrückende Bypaßleitung vorgesehen sind, um eine Leckprüfung des Dialysators vornehmen zu können.

Die WO 94/08641 beschreibt ein Überwachungssystem für eine Hämodialysevorrichtung mit einem Harnstoffsensor, der im Dialysierflüssigkeitsweg angeordnet ist. Die Druckschrift schlägt vor, den Eingang und Ausgang der Dialysierflüssigkeitskammer des Dialysators über einen Bypaß für eine vorbestimmte Zeitdauer von etwa 5 min. kurzzuschließen, so daß der Dialysierflüssigkeitsfluß durch die Dialysierflüssigkeitskammer unterbrochen ist. Obwohl der Dialysierflüssigkeitsfluß unterbrochen ist, soll die Ultrafiltration hingegen aufrechterhalten werden. Nach Ablauf der vorbestimmten Zeitdauer wird dann am Ausgang der Dialysierflüssigkeitskammer eine Probe genommen, die zur Bestimmung der Clearance des Dialysators herangezogen werden kann.

Der Erfindung liegt die Aufgabe zugrunde, ein Verfahren zur Bestimmung von Parametern der Hämodialyse anzugeben, das eine sehr einfache, dialysatseitige Messung der Konzentration einer am Stoffaustausch des Dialysators teilnehmenden Substanz im Blut als der zu bestimmende Parameter oder als Zwischenwert für den zu bestimmenden Parameter während der extrakorporalen Blutbehandlung erlaubt. Die Lösung dieser Aufgabe erfolgt erfindungsgemäß mit den im Patentanspruch 1 angegebenen Merkmalen.

Eine weitere Aufgabe der Erfindung liegt darin, eine Blutbehandlungsvorrichtung mit einer Einrichtung zur Bestimmung von Parametern der Hämodialyse zu schaffen, die eine einfache, dialysatseitige Messung einer am Stoffaustausch des Dialysators teilnehmenden Substanz im Blut als der zu bestimmende Parameter oder als Zwischenwert für den zu bestimmenden Parameter während der extrakorporalen Blutbehandlung erlaubt. Diese Aufgabe wird erfindungsgemäß mit den im Patentanspruch 13 angegebenen Merkmalen gelöst.

Es hat sich überraschenderweise gezeigt, daß die Konzentration einer Substanz in der aus der Dialysierflüssigkeitskammer des Dialysators strömenden Dialysierflüssigkeit im wesentlichen die Konzentration der betrachteten Substanz in dem in die Blutkammer strömenden Blut weitgehend unabhängig von den Behandlungsbedingungen annimmt, wenn die Flußrate der durch die Dialysierflüssigkeitskammer strömenden Dialysierflüssigkeit ausgehend von einem für die Blutbehandlung vorgegebenen Wert auf einen bestimmten Wert reduziert wird. Demnach ist es nicht erforderlich, zur Einstellung eines Gleichgewichtszustandes den Dialysierflüssigkeitsfluß durch die Dialysierflüssigkeitskammer zu unterbrechen. Trotz variabler Behandlungsbedingungen kann die Konzentration einer Substanz am Eingang der Blutkammer des Dialysators durch eine Messung der Konzentration der Substanz am Ausgang der Dialysierflüssigkeitskammer mit ausreichender Genauigkeit bestimmt werden, wenn die Dialysierflüssigkeitsrate während der extrakorporalen Blutbehandlung reduziert wird. Nach der Bestimmung der Bluteingangskonzentration, d.h. der Konzentration einer Substanz in dem in die Blutkammer des Dialysators strömenden Bluts, durch eine Messung der Dialysierflüssigkeitsausgangskonzentration, d.h. der Konzentration einer Substanz in der aus der Dialysierflüssigkeitskammer strömenden Dialysierflüssigkeit, als der zu bestimmende Parameter oder als Zwischenwert für den zu bestimmenden Parameter, kann die Dialysierflüssigkeitsrate auf einen für die Blutbehandlung vorgegebenen Wert eingestellt werden. Von Vorteil ist, daß nicht nur ein begrenztes Probenvolumen zur Verfügung steht, sondern die die Dialysierflüssigkeitskammer verlassende Flüssigkeit genau solange auf dem zu messenden Konzentratniveau gehalten werden kann, wie es für die Messung erforderlich ist. Eine andauernde Ultrafiltration zum Abziehen des Probenvolumens ist nicht notwendig, da der Meßzustand allein über den diffusiven Ausgleich zustande kommen kann. Damit kann das erfindungsgemäße Verfahren auch in Situationen angewendet werden, in denen eine Fortführung der Ultrafiltration zu einem kritischen Zustand des Patienten führen kann. Andererseits kann eine Ultrafiltration zur Unterstützung des Meßverfahrens jederzeit fortgeführt werden.

Von Vorteil ist auch, daß die Behandlung während des Meßverfahrens fortgeführt werden kann, wenn auch mit verminderter Effizienz. Weiterhin ist es vorteilhaft, daß der Dialysator bzw. die Membran auch während der Messung durch- bzw. freigespült wird. Dadurch wird verhindert, daß sich während der Messung Rückstände im Dialysator bilden.

Die Einstellung der Dialysierflüssigkeitsflußrate auf einen Wert, bei dem die Konzentration der Substanz in der Dialysierflüssigkeitskammer im wesentlichen die Konzentration derselben im Blut annimmt, kann zu Beginn, im Laufe oder zum Ende der Blutbehandlung erfolgen. Bei einer initialen Messung wird gleich die reduzierte Dialysierflüssigkeitsrate eingestellt, um danach auf den Behandlungswert erhöht zu werden. Während der Blutbehandlung wird die Dialysierflüssigkeitsrate ausgehend von einem für die Behandlung vorgegebenen Wert reduziert und nach der Messung auf den ursprünglichen Wert wieder erhöht.

Es hat sich gezeigt, daß bei einer Blutflußrate von > 300 ml/min die Dialysierflüssigkeitsrate nur auf einen Wert von < 100 ml/min eingestellt zu werden braucht, um einen Gleichgewichtszustand einzustellen, der eine Messung mit genügender Genauigkeit erlaubt.

Mit abnehmender Blutflußrate ist die Dialysierflüssigkeitsrate auf einen geringeren Wert einzustellen. Bei einer Blutflußrate von > 150 ml/min stellt sich ein ausreichendes Gleichgewicht ein, wenn die Dialysierflüssigkeitsrate < 75 ml/min ist.

In der Praxis hat sich überraschenderweise gezeigt, daß sich weitgehend unabhängig von den Behandlungsbedingungen, z.B. der Höhe des Blutflusses oder der Membranfläche des Dialysators immer dann ein ausreichendes Gleichgewicht einstellt, wenn die Dialysierflüssigkeitsrate auf einen Wert von 40 bis 60 ml/min, vorzugsweise 50 ml/min eingestellt wird. Nur in Ausnahmefällen kann es erforderlich sein, eine Dialysierflüssigkeitsflußrate von 10 bis 30 ml/min, vorzugsweise 20 ml/min, einzustellen.

Der vorgegebene Zeitraum für die Gleichgewichtseinstellung kann im Bereich von 1 bis 5 min. liegen. Die Einstellzeit kann aber auch durch weitere Maßnahmen, z.B. Druckstöße im Dialysierflüssigkeitsweg und/oder parallele Ultrafiltration, reduziert werden. Auch ist eine Extrapolation des Endwertes auf der Grundlage von Messungen in einem kürzeren Zeitraum möglich. Derartige Verfahren finden z.B. bei elektronischen Fieberthermometern Anwendung.

Im folgenden wird die Erfindung unter Bezugnahme auf die Zeichnungen näher erläutert.

Es zeigen:

Figur 1      ein Prinzipschaltbild einer Blutbehandlungsvorrichtung mit einer Einrichtung zur Bestimmung von Parametern der Hämodialyse,

Figur 2a      ein Schaubild, in dem das bei einem Dialysator mit einer Membranfläche von 0,7 $m^2$ gemessene Verhältnis von dialysatseitiger Leitfähigkeit und blutseitiger Leitfähigkeit als Funktion der Dialysierflüssigkeitsflußrate dargestellt ist,

Figur 2b      ein Schaubild, in dem das bei einem Dialysator mit einer Membranfläche von 1,8 $m^2$ gemessene Verhältnis von dialysatseitiger Leitfähigkeit und blutseitiger Leitfähigkeit als Funktion der Dialysierflüssigkeitsflußrate dargestellt ist und

Figur 2c      ein Schaubild, in dem die zeitliche Änderung der dialysatseitigen Leitfähigkeit infolge einer Veränderung der Dialysierflüssigkeitsflußrate dargestellt ist.

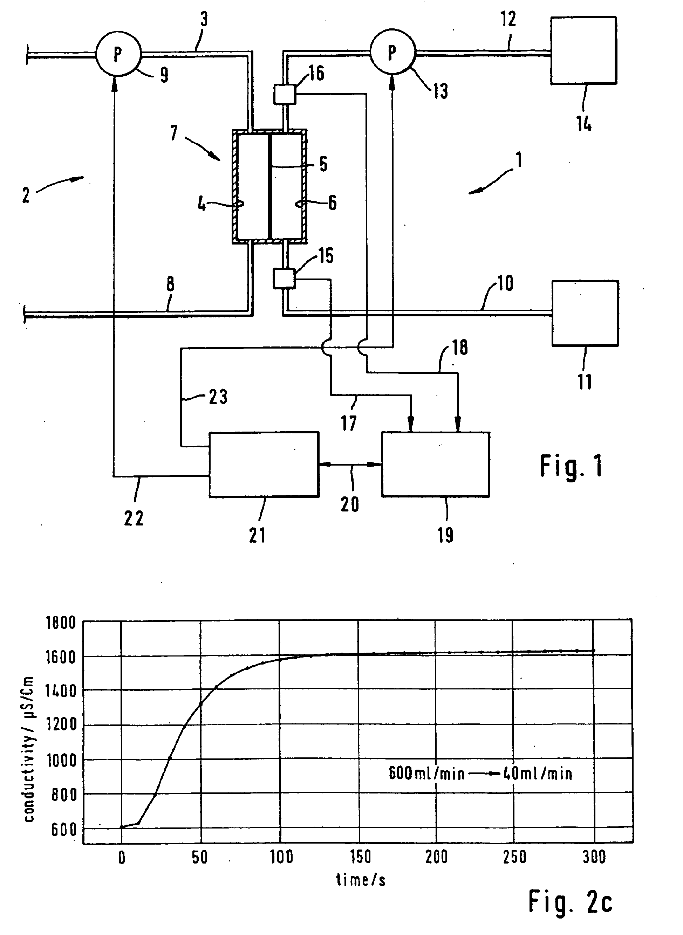

In Figur 1 sind nur die wesentlichen Komponenten einer Dialysevorrichtung dargestellt. Die Dialysevorrichtung besteht im wesentlichen aus einem Dialysierflüssigkeitsteil 1 und einem extrakorporalen Blutkreislauf 2. Der extrakorporale Blutkreislauf 2 umfaßt einen arteriellen Zweig 3, eine Blutkammer 4 eines durch eine semipermeable Membran 5 in die Blutkammer 4 und eine Dialysierflüssigkeitskammer 6 unterteilten Dialysators 7 und einen venösen Zweig 8. Im arteriellen Zweig 3 ist eine Blutpumpe 9 angeordnet, mit der eine bestimmte Blutflußrate Qb im extrakorporalen Kreislauf vorgegeben werden kann.

Die Dialysierflüssigkeitskammer 6 des Dialysators 7 ist über eine Zuführleitung 10 mit einer Dialysierflüssigkeitsquelle 11 verbunden. Eine Abführleitung 12, in die eine die Flußrate Qd der Dialysierflüssigkeit vorgebende Dialysierflüssigkeitspumpe 13 geschaltet ist, führt in einen Auslauf 14.

In der Zuführleitung 10 und der Abführleitung 12 ist jeweils eine Meßeinrichtung 15, 16 zur Bestimmung der Stoffkonzentration der Dialysierflüssigkeit am Eingang des Dialysators 7 und der Stoffkonzentration der Dialysierflüssigkeit am Ausgang des Dialysators angeordnet. Die Meßeinrichtung 15 ist zur Durchführung des erfindungsgemäßen Verfahrens zunächst nicht notwendig. Für die Bestimmung weiterer Parameter ist das Vorhandensein einer solche Meßeinrichtung jedoch vorteilhaft. Die Meßeinrichtungen 15, 16 zur Bestimmung der Dialysierflüssigkeitseingangs- und - ausgangskonzentration cdi bzw. cdo weisen stromauf und stromab des Dialysators 7 angeordnete Leitfähigkeitssensoren auf, die vorzugsweise die temperaturkorrigierte Leitfähigkeit der Dialysierflüssigkeit messen, die hauptsächlich durch die Na-Konzentration bedingt ist. Anstelle von Leitfähigkeitssensoren können auch optische Sensoren oder Enzymsensoren etc. zur Messung der Dialysierflüssigkeitskonzentration im Dialysierflüssigkeitsweg 1 angeordnet sein. Anstelle eines Leitfähigkeitssensors kann die Meßeinrichtung 16 stromab des Dialysators 7 auch einen Harnstoffsensor aufweisen, wobei ein Harnstoffsensor stromauf des Dialysators entfallen kann, wenn die Clearance bestimmt werden soll. Die Meßeinrichtungen 15, 16 sind über Datenleitungen 17, 18 mit einer Auswerteinheit 19 verbunden, in der die Ausgangssignale der Meßeinrichtungen verarbeitet werden. Die Auswerteinheit kommuniziert über eine Datenleitung 20 mit einer Steuereinheit 21. Die Steuereinheit 21 ist über Steuerleitungen 22, 23 mit der Blutpumpe 9 und der Dialysierflüssigkeitspumpe 13 verbunden, so daß die Blutflußrate Qb bzw. die Dialysierflüssigkeitsflußrate Qd eingestellt und variiert werden kann.

Während der Dialysebehandlung strömt die in der Dialysierflüssigkeitsquelle 11 bereitgestellte Dialysierflüssigkeit durch die Dialysierflüssigkeitskammer 6 des Dialysators 7 mit einer durch die Fördermenge der Dialysierflüssigkeitspumpe 13 vorgegebenen Flußrate Qd, während das zu behandelnde Blut des Patienten die Blutkammer 4 des Dialysators 7 mit einer durch die Blutpumpe 9 vorgegebenen Flußrate Qb durchströmt.

Der von der Steuereinheit 21 vorgegebene Programmablauf zur Bestimmung der Bluteingangskonzentration cbi wird nachfolgend erläutert.

Während der Hämodialysebehandlung wird die Dialysierflüssigkeitsflußrate auf 40 bis 60 ml/min, vorzugsweise 50 ml/min, reduziert. Die Reduzierung der Flußrate erfolgt dadurch, daß die Steuereinheit 21 die Dialysierflüssigkeitspumpe 13 entsprechend ansteuert. Nach Ablauf einer Zeitdauer von 2 bis 3 Minuten, die von einem in der Steuereinheit vorgesehenen Zeitglied vorgegeben wird, steuert die Steuereinheit die Auswerteinheit 19 derart an, daß die Auswerteinheit die mit der Meßeinrichtung 16 gemessene Dialysierflüssigkeitsausgangskonzentration cdo erfaßt. Die gemessene Dialysierflüssigkeitsausgangskonzentration cdo entspricht dann im wesentlichen der Bluteingangskonzentration cbi als den zu bestimmenden Parameter. Anschließend stellt die Steuereinheit 21 mit der Dialysierflüssigkeitspumpe 13 die Dialysierflüssigkeitsflußrate wieder auf den ursprünglichen Wert ein.

Die ermittelte Bluteingangskonzentration cbi kann als Zwischenwert für die Ermittlung weiterer Parameter herangezogen werden. So läßt sich die Dialysance des Dialysators 7 durch zwei weitere Messungen ermitteln. Hierzu steuert die Steuereinheit 21 die Auswerteinheit 19 derart an, daß diese die bei einem vorgegebenen Dialysierflüssigkeitsfluß Qd mit der Meßeinrichtung 15 gemessene Dialysierflüssigkeitseingangskonzentration cbi und die sich nunmehr einstellende Dialysierflüssigkeitsausgangskonzentration cdo, die mit der Meßeinrichtung 16 gemessen wird, erfaßt. In der Auswerteinheit wird aus der zuvor bestimmten Bluteingangskonzentration cdi und der Dialysierflüssigkeitseingangs- und -ausgangskonzentration cdi, cdo sowie der vorgegebenen Dialysierflüssigkeitsrate Qd nach der Gleichung

$$D = Qd \, \frac{(cdo - cdi)}{cbi - cdi} \qquad\qquad (3)$$

die Dialysance D bestimmt.

Die zu bestimmenden Parameter können auf einer nicht dargestellten Anzeigeeinheit ausgegeben bzw. zur weiteren Prozeßsteuerung herangezogen werden.

Das erfindungsgemäße Verfahren beruht darauf, daß bei einer Reduzierung der Flußrate der Dialysierflüssigkeit trotz variabler Behandlungsbedingungen die Bluteingangskonzentration dadurch bestimmt werden kann, daß die Dialysierflüssigkeitsausgangskonzentration gemessen wird. Dies hat den Vorteil, daß ein Eingriff in den extrakorporalen Blutkreislauf nicht erforderlich ist. Die Genauigkeit des Meßverfahrens hat sich in den nachfolgenden Experimenten bestätigt.

Es wurde mittels zweier Schlauchrollenpumpen der gekoppelte Dialysat- und Blutkreislauf nachgebildet. Da Diffusionsverhalten und Dialysator-Clearances für Elektrolyte und für Harnstoff nahezu identisch sind, konnte anstelle von Harnstoff NaCl als Testsubstanz verwendet werden. Dadurch ist über die Leitfähigkeitsmessung eine einfache, hochgenaue Methode zur Konzentrationsmessung verfügbar. Blutseitig wurde eine 15 millimolare NaCl-Lösung verwendet. Diese Konzentration liegt in der Größenordnung der typischen Harnstoffkonzentration. Dialysatseitig wurde reines Wasser verwendet. Die Leitfähigkeitsmeßzellen wurden stromauf und stromab der Dialysierflüssigkeitskammer des Dialysators angeordnet.

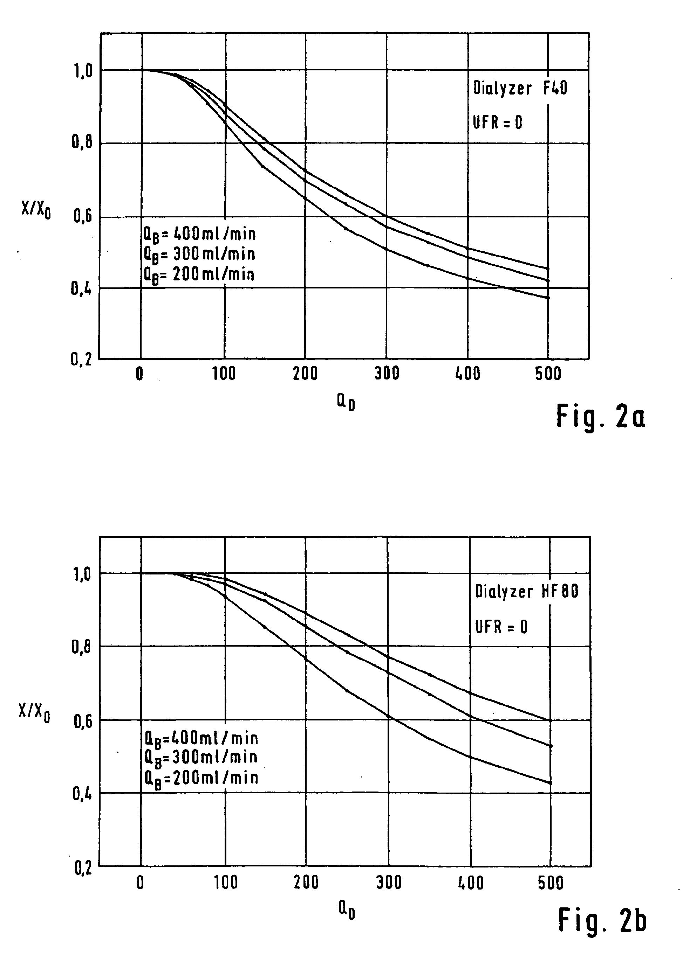

Die Messungen wurden mit verschiedenen Dialysatortypen durchgeführt, die sich in ihrer Membranfläche voneinander unterscheiden. Für jeden Dialysator wurden Meßkurven für einige Blutflüsse aufgenommen. Als minimaler Blutfluß, der klinisch üblich ist, wurde 200 ml/min angesetzt. Für einen gegebenen Blutfluß Qb wurde der Dialysatfluß Qd schrittweise von 500 ml/min bis 0 ml/min verändert, wobei nach jeder Veränderung des Dialysatflusses Qd die Einstellung eines stabilen Zustandes abgewartet und dann das Verhältnis der dialysatseitigen zur blutseitigen Leitfähigkeit bestimmt wurde. Figur 2a zeigt das Verhältnis $X/X_0$ der dialysatseitigen Leitfähigkeit zur blutseitigen Leitfähigkeit als Funktion der Dialysierflüssigkeitsflußrate Qd bei einem Dialysator mit einer Membranfläche von 0,7 m$^2$, während Figur 2b das Verhältnis von dialysatseitiger zur blutseitiger Leitfähigkeit als Funktion der Dialysierflüssigkeitsflußrate bei einem Dialysator mit einer Membranfläche von 1,8 m$^2$ zeigt. Die dialysatseitige Leitfähigkeit bzw. blutseitige Leitfähigkeit entspricht der Dialysierflüssigkeitsausgangs- bzw. Bluteingangskonzentration. Wie die Messungen zeigen, wird das Gleichgewicht bei einer Dialysierflüssigkeitsflußrate Qd von 50 ml/min nahezu vollständig erreicht. Bei dem Dialysator mit einer Membranfläche von 0, 7 m$^2$ ist das Verhältnis $X/X_0 > 0,97$ für Qb $\geq$ 200 ml/min und bei einem Dialysator mit einer Membranfläche von 1,8 m$^2$ ist $X/X_0 > 0,99$ für Qb $\geq$ 200 ml/min. Bei höheren Blutflüssen ergibt sich ein noch besserer Konzentrationsausgleich.

**Patentansprüche**

1. Verfahren zur Bestimmung von Parametern der Hämodialyse während einer extrakorporalen Blutbehandlung, bei der das zu behandelnde Blut in einem extrakorporalen Blutkreislauf die Blutkammer eines durch eine semipermeable Membran in die Blutkammer und eine Dialysierflüssigkeitskammer unterteilten Dialysators mit einer vorgegebenen Blutflußrate durchströmt und Dialysierflüssigkeit in einem Dialysierflüssigkeitsweg die Dialysierflüssigkeitskammer des Dialysators mit einer vorgegebenen Dialysierflüssigkeitsflußrate durchströmt, mit folgenden Verfahrensschritten:

   Einstellen der Dialysierflüssigkeitsflußrate Qd auf einen Wert, bei dem die Konzentration cdo einer Substanz in der aus der Dialysierflüssigkeitskammer strömenden Dialysierflüssigkeit im wesentlichen die Konzentration cbi der betrachteten Substanz in dem Blut annimmt, das in die Blutkammer strömt, und
   Messen der Konzentration der Substanz in der aus der Dialysierflüssigkeitskammer strömenden Dialysierflüssigkeit als der zu bestimmende Parameter oder als Zwischenwert für den zu bestimmenden Parameter.

2. Verfahren nach Anspruch 1, **dadurch gekennzeichnet, daß** die Dialysierflüssigkeitsrate Qd zum Ende der Blutbehandlung ausgehend von einem für die Blutbehandlung vorgegebenen Wert auf den Wert reduziert wird, bei dem die Konzentration cdo der Substanz in der Dialysierflüssigkeit im wesentlichen die Konzentration cbi derselben im Blut annimmt.

3. Verfahren nach Anspruch 1, **dadurch gekennzeichnet, daß** die Dialysierflüssigkeitsrate Qd zu Beginn der Blutbehandlung auf den Wert eingestellt wird, bei dem die Konzentration cdo der Substanz in der Dialysierflüssigkeit im wesentlichen die Konzentration cbi derselben im Blut annimmt, und nach dem Messen der Konzentration der Substanz in der Dialysierflüssigkeit auf einen für die Blutbehandlung vorgegebenen Wert erhöht wird.

4. Verfahren nach Anspruch 1, **dadurch gekennzeichnet, daß** die Dialysierflüssigkeitsrate Qd während der Blutbehandlung ausgehend von einem für die Blutbehandlung vorgegebenen Wert auf den Wert reduziert wird, bei dem die Konzentration cdo der Substanz in der Dialysierflüssigkeit im wesentlichen die Konzentration cbi derselben im Blut annimmt, und nach dem Messen der Konzentration der Substanz in der Dialysierflüssigkeit wieder auf den ursprünglichen Wert erhöht wird.

5. Verfahren nach einem der Ansprüche 1 bis 4, **dadurch gekennzeichnet, daß** die Konzentration cdo der Substanz in der Dialysierflüssigkeit nach der Einstellung des Gleichgewichtszustandes gemessen wird.

6. Verfahren nach Anspruch 5, **dadurch gekennzeichnet, daß** der vorgegebene Zeitraum für die Gleichgewichtseinstellung im Bereich von 1 - 5 min liegt.

7. Verfahren nach einem der Ansprüche 1 bis 6, **dadurch gekennzeichnet, daß** die Dialysierflüssigkeitsflußrate Qd bei einer Blutflußrate Qb von > 300 ml/min auf einen Wert von < 100 ml/min eingestellt wird.

8. Verfahren nach einem der Ansprüche 1 bis 6, **dadurch gekennzeichnet, daß** die Dialysierflüssigkeitsflußrate Qd bei einer Blutflußrate Qb von > 150 ml/min auf einen Wert von < 75 ml/min eingestellt wird.

9. Verfahren nach einem der Ansprüche 1 bis 6, **dadurch gekennzeichnet, daß** die Dialysierflüssigkeitsflußrate Qd auf einen Wert von 40 - 60 ml/min, vorzugsweise 50 ml/min eingestellt wird.

10. Verfahren nach einem der Ansprüche 1 bis 6, **dadurch gekennzeichnet, daß** die Dialysierflüssigkeitsflußrate Qd auf einen Wert von 10 - 30 ml/min, vorzugsweise 20 ml/min eingestellt wird.

11. Verfahren nach einem der Ansprüche 4 bis 10, **dadurch gekennzeichnet, daß** nach dem Erhöhen der Dialysierflüssigkeitsrate Qd auf den ursprünglichen Wert bei der vorgegebenen Blutflußrate Qb und der vorgegebenen Dialysierflüssigkeitsrate die Konzentration cdi der Substanz in der in die Dialysierflüssigkeitskammer strömenden Dialysierflüssigkeit und die Konzentration cdo der Substanz in der aus der Dialysierflüssigkeitskammer strömenden Dialysierflüssigkeit gemessen werden, und nach der Gleichung

$$D = Qd\,\frac{(cdo - cdi)}{cbi - cdi} \quad (3)$$

die Dialysance D als Parameter bestimmt wird.

12. Verfahren nach einem der Ansprüche 1 bis 11, **dadurch gekennzeichnet, daß** die Konzentrationsmessung durch eine Leitfähigkeitsmessung erfolgt.

13. Blutbehandlungsvorrichtung mit
einem Dialysator (7), der eine in einen extrakorporalen Blutkreislauf (2) geschaltete Blutkammer (4) aufweist, die durch eine semipermeable Membran (5) von einer Dialysierflüssigkeitskammer (6) getrennt ist, die in einen Dialysierflüssigkeitsweg (1) geschaltet ist, wobei in den extrakorporalen Blutkreislauf eine Blutpumpe (9) und in den Dialysierflüssigkeitsweg eine Dialysierflüssigkeitspumpe (13) geschaltet ist, und
einer Einrichtung zur Bestimmung von Parametern der Hämodialyse während der extrakorporalen Blutbehandlung,
**dadurch gekennzeichnet, daß**
die Einrichtung zur Bestimmung von Parametern der Hämodialyse aufweist:

eine im Dialysierflüssigkeitsweg (1) stromab der Dialysierflüssigkeitskammer (6) angeordnete Meßeinrichtung (16) zum Messen der Konzentration cdo der Substanz in der aus der Dialysierflüssigkeitskammer strömengen Dialysierflüssigkeit,
einer Steuereinheit (21), die derart ausgebildet ist, daß die Fördermenge der Dialysierflüssigkeitspumpe (13) während der Dialysebehandlung von einem vorgegebenen Wert für eine vorgegebene Zeitdauer auf einen Wert reduzierbar ist, bei dem die mit der Meßeinrichtung (16) gemessene Konzentration cdo der Substanz in der aus der Dialysierflüssigkeitskammer strömenden Dialysierflüssigkeit im wesentlichen die Konzentration cbi der betrachteten Substanz in dem Blut annimmt, das in die Blutkammer (4) strömt und
eine das Ausgangssignal der Meßeinrichtung (16) empfangene Auswerteinheit (19), die von der Steuereinheit (21) derart gesteuert ist, daß die Auswerteinheit die Konzentration cdo der betrachteten Substanz als der zu bestimmende Parameter oder als Zwischenwert für den zu bestimmenden Parameter erfaßt, bevor die Steuereinheit die Fördermenge auf den ursprünglichen Wert erhöht.

14. Blutbehandlungsvorrichtung nach Anspruch 13, **dadurch gekennzeichnet, daß** die Einrichtung zur Bestimmung von Parametern der Hämodialyse eine zweite im Dialysierflüssigkeitsweg (1) stromauf der Dialysierflüssigkeitskammer (6) angeordnete Meßeinrichtung (15) zum Messen der Konzentration cdi der Substanz in der in die Dialysierflüssigkeitskammer strömenden Dialysierflüssigkeit aufweist, deren Ausgangssignal die Auswerteinheit (19) empfängt, wobei die Auswerteinheit von der Steuereinheit (21) derart gesteuert ist, daß die Auswerteinheit die Konzentration cdi der betrachteten Substanz in der in die Dialysierflüssigkeitskammer (6) strömenden Dialysierflüssigkeit und die Konzentration cdo der Substanz in der aus der Dialysierflüssigkeitskammer strömenden Dialysierflüssigkeit nach Ablauf der vorgegebenen Zeitdauer erfaßt, wenn die Steuereinheit die Fördermenge auf den ursprünglichen Wert erhöht hat und daß die Auswerteinheit (19) derart ausgebildet ist, daß sie bei der vorgegebenen Blutflußrate Qb und der vorgegebenen Dialysierflüssigkeitsrate Qd sowie der gemessenen Konzentration cdi der Substanz in der in die Dialysierflüssigkeitskammer strömenden Dialysierflüssigkeit und der Konzentration cdo der Substanz in der aus der Dialysierflüssigkeitskammer strömenden Dialysierflüssigkeit die Auswerteinheit nach der Gleichung

$$D = Qd\ \frac{(cdo - cdi)}{cbi - cdi} \qquad (3)$$

die Dialysance D bestimmt.

## Claims

1. A method for determining parameters of the haemodialysis during an extracorporeal blood treatment, wherein the blood to be treated in an extracorporeal blood circuit flows at a preset blood flow rate through the blood chamber of a dialyser divided by a semi-permeable membrane into the blood chamber and a dialysing fluid chamber and dialysing fluid in a dialysing fluid path flows at a preset dialysing-fluid rate through the dialysing fluid chamber of the dialyser, with the following process steps:

   adjustment of dialysing fluid flow rate Qd to a value at which concentration cdo of a substance in the dialysing fluid flowing out of the dialysing fluid chamber essentially assumes concentration cbi of the considered substance in the blood which is flowing into the blood chamber, and
   measurement of the concentration of the substance in the dialysing fluid flowing out of the dialysing fluid chamber as the parameter to be determined or as an intermediate value for the parameter to be determined.

2. The method according to claim 1, **characterised in that** dialysing fluid rate Qd at the end of the blood treatment is reduced, starting from a value preset for the blood treatment, to a value at which concentration cdo of the substance in the dialysing fluid essentially assumes concentration cbi thereof in the blood.

3. The method according to claim 1, **characterised in that** dialysing fluid rate Qd is adjusted at the start of the blood treatment to the value at which concentration cdo of the substance in the dialysing fluid essential assumes concentration cbi thereof in the blood and, after the measurement of the concentration of the substance in the dialysing fluid, is increased to a value preset for the blood treatment.

4. The method according to claim 1, **characterised in that** dialysing fluid rate Qd is reduced during the blood treatment, starting from a value preset for the blood treatment, to the value at which concentration cdo of the substance in the dialysing fluid essentially assumes concentration cbi thereof in the blood and, after the measurement of the concentration of the substance in the dialysing fluid, is increased again to the original value.

5. The method according to any one of claims 1 to 4, **characterised in that** concentration cdo of the substance in the dialysing fluid is measured after adjustment of the equilibrium state.

6. The method according to claim 5, **characterised in that** the preset period for the equilibrium adjustment lies in the range from 1-5 min.

7. The method according to any one of claims 1 to 6, **characterised in that** dialysing fluid flow rate Qd is adjusted to a value of < 100 ml/min with a blood flow rate Qb > 300 ml/min.

8. The method according to any one of claims 1 to 6, **characterised in that** dialysing fluid flow rate Qd is adjusted to a value of < 75 ml/min with a blood flow rate Qb > 150 ml/min.

9. The method according to any one of claims 1 to 6, **characterised in that** dialysing fluid flow rate Qd is adjusted to a value of 40 - 60 ml/min, preferably 50 ml/min.

10. The method according to any one of claims 1 to 6, **characterised in that** dialysing fluid flow rate Qd is adjusted to a value of 10 - 30 ml/min, preferably 20 ml/min.

11. The method according to any one of claims 4 to 10, **characterised in that**, after the increase in dialysing fluid rate Qd to the original value with preset blood flow rate Qb and the preset dialysing fluid rate, concentration cdi of the substance in the dialysing fluid flowing into the dialysing fluid chamber and concentration cdo of the substance in the dialysing fluid flowing out of the dialysing fluid chamber are measured, and dialysance D is determined as a parameter according to the equation

$$D = Qd \frac{(cdo - cdi)}{cbi - cdi} \qquad (3)$$

12. The method according to any one of claims 1 to 11, **characterised in that** the concentration measurement is carried out by means of a conductivity measurement.

13. A blood treatment apparatus with
a dialyser (7), which has a blood chamber (4) incorporated into an extracorporeal blood circuit (2), said blood chamber being separated by a semi-permeable membrane (5) from a dialysing fluid chamber (6), which is incorporated into a dialysing fluid path (1), a blood pump (9) being incorporated in the extracorporeal blood circuit and a dialysing fluid pump (13) being incorporated in the dialysing fluid path, and
a device for determining parameters of the haemodialysis during the extracorporeal blood treatment,
**characterised in that**
the device for determining parameters of the haemodialysis comprises:

    a measuring device (16) disposed in the dialysing fluid path (1) downstream of the dialysing fluid chamber (6) for measuring concentration cdo of the substance in the dialysing fluid flowing out of the dialysing fluid chamber,
    a control unit (21), which is designed in such a way that the delivery quantity of the dialysing fluid pump (13) during the dialysis treatment can be reduced from a preset value for a preset period to a value at which concentration cdo of the substance measured with the measuring device (16) in the dialysing fluid flowing out of the dialysing fluid chamber essentially assumes concentration cbi of the considered substance in the blood which is flowing into the blood chamber (4) and
    an evaluation unit (19) receiving the output signal of the measuring device (16), said evaluation unit being controlled by the control unit (21) in such a way that the evaluation unit detects concentration cdo of the considered substance as the parameter to be determined or as an intermediate value for the parameter to be determined, before the control unit increases the delivery quantity to the original value.

14. The blood treatment apparatus according to claim 13, **characterised in that** the device for determining parameters of the haemodialysis comprises a second measuring device (15) disposed in the dialysing fluid path (1) upstream of the dialysing fluid chamber (6) for measuring concentration cdi of the substance in the dialysing fluid flowing into the dialysing fluid chamber, the output signal of which measuring device being received by the evaluation unit (19), the evaluation unit being controlled by the control unit (21) in such a way that the evaluation unit detects concentration cdi of the considered substance in the dialysing fluid flowing into the dialysing fluid chamber (6) and concentration cdo of the substance in the dialysing fluid flowing out of the dialysing fluid chamber after the lapse of the preset period when the control unit has increased the delivery quantity to the original value, and that the evaluation unit (19) is designed in such a way that, at preset blood flow rate Qb and preset dialysing fluid rate Qd as well as measured concentration cdi of the substance in the dialysing fluid flowing into the dialysing fluid chamber and concentration cdo of the substance in the dialysing fluid flowing out of the dialysing fluid chamber, the evaluation unit determines dialysance D according to the equation

$$D = Qd \frac{(cdo - cdi)}{cbi - cdi} \qquad (3)$$

**Revendications**

1. Procédé de détermination de paramètres d'hémodialyse pendant un traitement du sang extracorporel, au cours duquel le sang à traiter s'écoule avec un débit sanguin prédéfini dans un circuit sanguin extracorporel à travers la chambre à sang d'un dialyseur subdivisé en une membrane semi-perméable dans la chambre à sang et en une chambre à liquide de dialyse et le liquide de dialyse s'écoule avec un débit de liquide de dialyse prédéfini dans une voie de liquide de dialyse à travers la chambre de liquide de dialyse du dialyseur, comprenant les étapes de procédé suivantes consistant à :

   ♦ régler le débit du liquide de dialyse Qd sur une valeur avec laquelle la concentration cdo d'une substance dans le liquide de dialyse s'écoulant hors de la chambre de liquide de dialyse adopte essentiellement la concentration cbi de la substance considérée dans le sang qui s'écoule dans la chambre à sang, et
   ♦ mesurer la concentration de la substance dans le liquide de dialyse s'écoulant hors de la chambre de liquide de dialyse comme paramètre à déterminer et comme valeur intermédiaire pour le paramètre à déterminer.

2. Procédé selon la revendication 1, **caractérisé en ce que** le débit du liquide de dialyse Qd est réduit à la fin du traitement du sang, sur la base d'une valeur prédéfinie pour le traitement du sang, à la valeur pour laquelle la concentration cdo de la substance dans le liquide de dialyse adopte essentiellement la concentration cbi de celle-ci dans le sang.

3. Procédé selon la revendication 1, **caractérisé en ce que** le débit du liquide de dialyse Qd est réglé pour commencer le traitement de sang, sur la valeur pour laquelle la concentration cdo de la substance dans le liquide de dialyse adopte essentiellement la concentration cbi de celle-ci dans le sang, et il cet accru après la mesure de la concentration de la substance dans le liquide de dialyse jusqu'à une valeur prédéfinie pour le traitement de sang.

4. Procédé selon la revendication 1, **caractérisé en ce que** le débit du liquide de dialyse Qd est réduit pendant le traitement de sang, sur la base d'une valeur prédéfinie pour le traitement de sang, à la valeur pour laquelle la concentration cdo de la substance dans le liquide de dialyse adopte essentiellement la concentration cbi de celle-ci dans le sang, et il est augmenté à nouveau après la mesure de la concentration de la substance dans le liquide de dialyse jusqu'à la valeur d'origine.

5. Procédé selon l'une quelconque des revendications 1 à 4, **caractérisé en ce que** la concentration cdo de la substance dans le liquide de dialyse est mesurée après le réglage de l'état d'équilibre.

6. Procédé selon la revendication 5, **caractérisé en ce que** la période prédéfinie pour le réglage de l'état d'équilibre se situe dans une plage comprise entre 1 et 5 mn.

7. Procédé selon l'une quelconque des revendications 1 à 6, **caractérisé en ce que** le débit du liquide de dialyse Qd est réglé sur une valeur < 100 ml/mn pour un débit sanguin Qb > 300 ml/mn.

8. Procédé selon l'une quelconque des revendications 1 à 6, **caractérisé en ce que** le débit du liquide de dialyse Qd est réglé sur une valeur < 75 ml/mn, pour un débit sanguin Qb > 150 ml/mn.

9. Procédé selon l'une quelconque des revendications 1 à 6, **caractérisé en ce que** le débit du liquide de dialyse Qd est réglé sur une valeur de 40 à 60 ml/mn, de préférence 50 ml/mn.

10. Procédé selon l'une quelconque des revendications 1 à 6, **caractérisé en ce que** le débit du liquide de dialyse Qd est réglé sur une valeur de 10 à 30 ml/mn, de préférence 20 ml/mn.

11. Procédé selon l'une quelconque des revendications 4 à 10, **caractérisé en ce que**, après avoir augmenté le débit du liquide de dialyse Qd jusqu'à une valeur d'origine pour le débit sanguin prédéfini Qb et le débit de liquide de dialyse prédéfini, la concentration cdi de la substance dans le liquide de dialyse s'écoulant dans la chambre de liquide de dialyse et la concentration cdo de la substance dans le liquide de dialyse s'écoulant hors de la chambre de liquide de dialyse sont mesurées, et la capacité dialysable D est déterminée comme paramètre selon l'équation suivante :

$$D = Qd\,\frac{(cdo - cdi)}{cbi - cdi} \qquad (3)$$

12. Procédé selon l'une quelconque des revendications 1 à 11, **caractérisé en ce que** la mesure de concentration est effectuée par une mesure de conductivité.

13. Dispositif de traitement de sang comprenant

- ♦ un dialyseur (7), qui comprend une chambre à sang (4) raccordée à un circuit sanguin (2) extracorporel, laquelle chambre à sang (4) est séparée par une membrane (5) semi-perméable d'une chambre de liquide de dialyse (6), qui est raccordée à une voie de liquide de dialyse (1), une pompe à sang (9) étant raccordée dans le circuit sanguin extracorporel et une pompe à liquide de dialyse (13) étant raccordée dans la voie du liquide de dialyse , et
- ♦ un dispositif de détermination des paramètres d'hémodialyse pendant le traitement du sang extracorporel,

**caractérisé en ce que** le dispositif de détermination des paramètres d'hémodialyse comprend :

- ♦ un dispositif de mesure (16) disposé dans la voie du liquide de dialyse (1) en aval de la chambre de liquide de dialyse (6) pour mesurer la concentration cdo de la substance dans le liquide de dialyse s'écoulant hors de la chambre de liquide de dialyse,
- ♦ une unité de commande (21) qui est conçue de telle manière que le débit de la pompe à liquide de dialyse (13) peut être réduit, pendant le traitement par dialyse, d'une valeur prédéfinie pendant une durée prédéfinie à une valeur pour laquelle la concentration cdo de la substance mesurée avec le dispositif de mesure (16) adopte essentiellement, dans le liquide de dialyse s'écoulant hors de la chambre de liquide de dialyse, la concentration cbi de la substance considérée dans le sang qui s'écoule dans la chambre à sang (4) et
- ♦ une unité d'interprétation (19) recevant le signal de sortie du dispositif de mesure (16) qui est commandée par l'unité de commande (21) de façon à ce que l'unité d'interprétation détecte la concentration cdo de la substance considérée comme paramètre à déterminer ou comme paramètre intermédiaire pour le paramètre à déterminer, avant que l'unité de commande n'augmente le débit pour atteindre la valeur d'origine.

14. Dispositif de traitement du sang selon la revendication 13, **caractérisé en ce que** le dispositif de détermination des paramètres d'hémodialyse comprend un deuxième dispositif de mesure (15) disposé dans la voie du liquide de dialyse (1) en amont de la chambre de liquide de dialyse (6) pour mesurer la concentration cdi de la substance dans le liquide de dialyse s'écoulant dans la chambre de liquide de dialyse, l'unité d'interprétation (19) recevant le signal de sortie du liquide de dialyse, moyennant quoi l'unité d'évaluation du module de commande (21) est commandée de telle manière que l'unité d'interprétation détecte la concentration cdi de la substance considérée dans le liquide de dialyse s'écoulant dans la chambre de liquide de dialyse (6) et la concentration cdo de la substance dans le liquide de dialyse s'écoulant hors de la chambre de liquide de dialyse une fois la durée prédéfinie écoulée, si l'unité de commande a augmenté le débit pour atteindre la valeur d'origine et si l'unité d'interprétation (19) est conçue de telle manière que, pour le débit sanguin prédéfini Qb et le débit de liquide de dialyse prédéfini Qd ainsi que pour la concentration cdi de la substance dans le liquide de dialyse s'écoulant dans la chambre de liquide de dialyse et pour la concentration cdo de la substance dans le liquide de dialyse s'écoulant hors de la chambre de liquide de dialyse, l'unité d'interprétation détermine la capacité dialysable D selon l'équation suivante :

$$D = Qd\,\frac{(cdo - cdi)}{cbi - cdi} \qquad (3)$$

Fig. 1

Fig. 2c

Fig. 2a

Fig. 2b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0291421 B1
- DE 3938662 C2
- EP 0428927 A1
- EP 0330892 A1
- EP 0547025 A
- US 5110477 A
- WO 9409351 A
- EP 0578585 B1
- WO 9408641 A

**In der Beschreibung aufgeführte Nicht-Patentliteratur**

- Principles and biophysics of dialysis. **SARGENT,, J.A. ; GOTCH. F.A.** Replacement of Renal Function by Dialysis. 1983
- **NGUYEN-KHOA MAN et al.** Herausgeber: Artificial Organs. Raven Press, 1985, vol. 9, 150-154